# EUROPEAN PATENT APPLICATION

(11) **EP 1 488 810 A1**
(43) Date of publication of application: **22.12.2004**
(21) Application number: 03744532.7
(22) Date of filing: 20.03.2003
(51) Int. Cl.: A61K 47/08, A61K 47/10, A61K 45/00, A61P 17/00, A61P 31/10, A61K 31/4164

(54) **PERCUTANEOUS ABSORPTION PROMOTERS AND COMPOSITIONS FOR TREATING ATHLETE'S FOOT**

(30) Priority: 20.03.2002 JP 2002077908
(71) Applicant: KANEKA CORPORATION, Osaka-shi, Osaka 530-8288 (JP)
(72) Inventor: FUJII, Kenji, Kobe-shi, Hyogo 651-1202 (JP); KAWABE, Taizo, Himeji-shi, Hyogo 672-8044 (JP); HOSOE, Kazunori, Takasago-shi, Hyogo 676-0025 (JP); HIDAKA, Takayoshi, Kobe-shi, Hyogo 655-0006 (JP)
(74) Representative: HOFFMANN - EITLE
(86) International application number: PCT/JP2003/003390
(87) International publication number: WO 2003/077951

(57) **Abstract**

The present invention provides to intractable skin diseases such as athlete's foot a substance which has a percutaneous absorption promoting effect capable of enhancing the effectiveness of an antifungal agent, etc., and is high in safety to skin which becomes sensitive to irritation because of the diseases.

Coenzyme Q, which is known to be highly safe in oral administration, was found to have high safety to skin and a percutaneous absorption promoting effect to an antifungal agent. From this, a useful composition composed of coenzyme Q and an antifungal agent can be provided to athlete's foot which is difficult to be completely cured.

## Description

### TECHNICAL FIELD

The present invention relates to an agent for promoting percutaneous absorption and a composition for treating athlete's foot comprising coenzyme Q.

### BACKGROUND ART

A percutaneous administration dosage form aiming at topical administration is advantageous to a disease having a shallow focus in that a drug concentration can be maintained high while reducing influences to the whole body, since the administration part is topical.

For example, indomethacin, which is an analgesic and anti-inflammation drug, is reported to have a side effect of stomach disorder. But the effect of indomethacin could be exerted without causing stomach disorder by carrying out topical administration. However, since skin originally functions as a barrier to an external environment, the osmolarity resistance of a corneum, etc. of skin is high. Accordingly, in the percutaneous administration dosage form, whether a drug can sufficiently be absorbed percutaneously becomes an important factor for exerting its effect.

As the most remarkable example thereof, there may be mentioned an agent for treating athlete's foot. Athlete's foot is a disease developed when a fungus proliferates in skin. In many athlete's foot drugs, an antifungal agent having a killing action on a fungus is used. However, although an antifungal agent shows strong sterilization effect to a fungus in vitro, to actual athlete's foot, temporary improvement is shown but complete cure cannot be attained in many cases. As a reason for that, there may be mentioned the fact that an antifungal agent cannot fully exert its effect to a fungus proliferating inside skin. That is, since the sufficient amount of antifungal agent cannot be infiltrated into skin because of a barrier of skin, the sterilization effect to a fungus is insufficient.

Herein, an agent for promoting percutaneous absorption with an action of promoting percutaneous absorption of a drug becomes to serve an important role. If it becomes possible to liberate a larger amount of antifungal agent on a fungus in skin by combinedly using an antifungal agent and an agent for promoting percutaneous absorption, the fungus is completely sterilized, and complete cure of athlete's foot may be possible. As such an agent for promoting percutaneous absorption, there have been known azone, phosphatides, terpenes, etc. However, azone is highly irritative to skin, and causes a problem on actual use in many cases. Not only for athlete's foot, but also for skin diseases in general, skin becomes sensitive to irritation in many cases. Thus, a drug irritative to skin is not desirable. Similarly, terpenes are also irritative to skin as represented by menthol. Since phosphatide is high in the combination effect with a base, it is difficult to find the most appropriate dosage form for respective drugs, thereby is low in versatility. As described above, the existing agents for promoting percutaneous absorption have many problems in use, thus an agent for promoting percutaneous absorption which is lowly irritative to skin, and capable of infiltrating a drug to skin has been asked for.

Coenzyme Q is an indispensable component widely distributed in living bodies from bacteria to mammals, and is known as an electronic transfer system component of mitochondria in cells of living bodies. Coenzyme Q serves as a transfer component in an electron transfer system by repeating oxidization and reduction cycles in mitochondria. In addition, reduced coenzyme Q is known to have an antioxidant effect. In humans, coenzyme Q₁₀, in which a side chain of coenzyme Q has 10 repeating units, is a main component.

As an important feature of coenzyme Q₁₀, its high level of safety may be mentioned. In the chronic toxicity test on a rat, it was reported that completely no toxic influence had been observed by everyday administration at the dosage of 1200 mg/kg/day for 52 weeks (K. D. Williams et al., J. Agric. Food Chem., 47, 3756-3763, 1999).

As coenzyme Q₁₀, oxidized coenzyme Q₁₀ is widely used as a congestive heart failure drug in Japan, and as a healthy food in Europe and the Unites States. Additionally, in recent years, the application as cosmetics has also become known. However, the function of coenzyme Q₁₀ in the above-mentioned application is energy supply by mitochondrial activation effect which is widely known, and an antioxidant effect by reduced coenzyme Q₁₀. Thus, it has been completely unknown that coenzyme Q₁₀ has a percutaneous absorption promoting effect to a drug. Furthermore, it has also been completely unknown that coenzyme Q₁₀ has a significant enhancing activity for the effect for treating athlete's foot by combinational use with an antifungal agent.

### SUMMARY OF THE INVENITON

The present invention has for its object to provide a composition for skin which is excellent in percutaneous absorption promoting effect, and thereby showing a strong effect for treating athlete's foot.

The present inventors have investigated to solve the above-mentioned subject. As a result, they found that oxidized coenzyme Q and reduced coenzyme Q have a promoting effect for percutaneous absorption of an antifungal agent, and are not irritative to skin.

That is, the present invention relates to
an agent for promoting percutaneous absorption
which comprises oxidized coenzyme Q represented by the following formula (1) 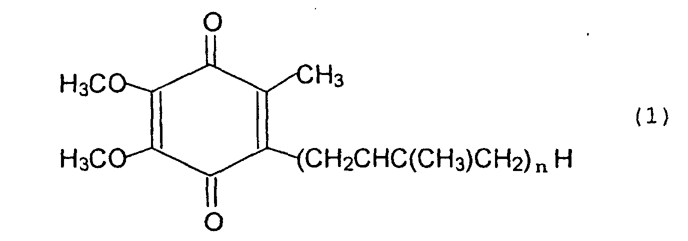 (wherein n represents an integer of 1 to 12) and/or reduced coenzyme Q represented by the following formula (2) 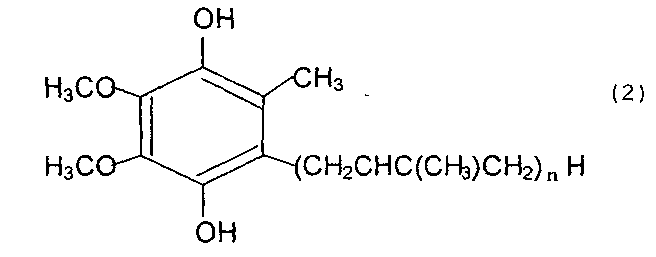 (wherein n represents an integer of 1 to 12) as an active ingredient.

The present invention also relates to
the agent for promoting percutaneous absorption, wherein coenzyme Q is coenzyme Q₁₀.

Furthermore, the present invention relates to
a composition for percutaneous administration
which comprises the agent for promoting percutaneous absorption; a composition for treating athlete's foot
which comprises the agent for promoting percutaneous absorption and an antifungal agent; and
a dermatologic treatment method
which comprises combinedly using the composition for percutaneous administration and a dermatologic composition.

### DETAILED DESCRIPTION OF THE INVENTION

Coenzyme Q is represented by the following formula (1) 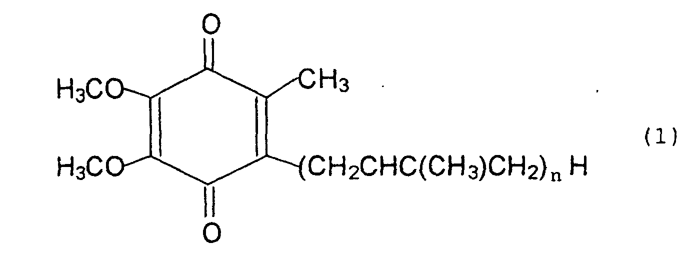 (wherein n represents an integer of 1 to 12), and the following formula (2) 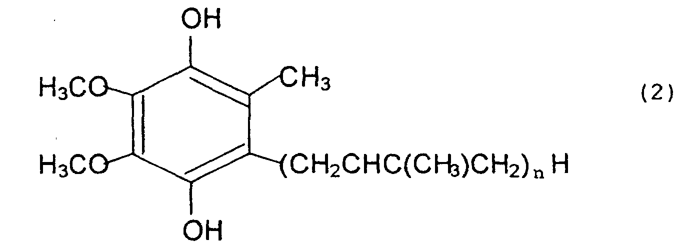 (wherein n represents an integer of 1 to 12). The formula (1) represents oxidized coenzyme Q, and the formula (2) represents reduced coenzyme Q.

The method for producing oxidized coenzyme Q and reduced coenzyme Q is not particularly restricted. For example, a method may be used which comprises producing coenzyme Q in the conventional manner such as synthesis, fermentation, or extraction from natural products, and concentrating an oxidized coenzyme Q₁₀-containing eluate fraction or a reduced coenzyme Q₁₀-containing eluate fraction by using chromatography, and the like method. When producing oxidized coenzyme Q, conventional methods may be used. When producing reduced coenzyme Q, a method may be used which comprises adding a general reducing agent such as sodium borohydride or sodium dithionite (sodium hydrosulfite) to the above-mentioned coenzyme Q where necessary, reducing the above-mentioned coenzyme Q to reduced coenzyme Q by a conventional manner, and concentrating the resultant with chromatography. Moreover, reduced coenzyme Q may also be obtained by a method comprising reacting existing high-purity coenzyme Q with the above-mentioned reducing agent.

As the coenzyme Q to be used in the present invention, there may be mentioned one having 1 to 12 repeating units (n in the formula) in the side chain as represented by the above-mentioned formula (1) and (2). Among them, one having 10 repeating units, i.e. coenzyme Q₁₀ is particularly preferably used.

The agent for promoting percutaneous absorption of the present invention comprises the above-mentioned oxidized coenzyme Q and/or the above-mentioned reduced coenzyme Q as an active ingredient.

The method for obtaining said agent for promoting percutaneous absorption is not particularly restricted. For example, a method may be used which comprises dissolving oxidized coenzyme Q and/or reduced coenzyme Q obtained as above in an appropriate solvent which is used in general such as isopropyl alcohol, acetone, and ether, to obtain thereby an agent for promoting percutaneous absorption containing the above-mentioned oxidized coenzyme Q and/or reduced coenzyme Q in a desired amount. Additionally, the above-mentioned oxidized coenzyme Q and the above-mentioned reduced coenzyme Q may simply be mixed in solids. Moreover, a mixture composed of oxidized coenzyme Q and reduced coenzyme Q obtained in the above-mentioned process for producing coenzyme Q may be used as such. Furthermore, the agent for promoting percutaneous absorption of the present invention may also directly be obtained by controlling time of the reduction reaction of the above-mentioned existing high-purity coenzyme Q and the species or amount of the reducing agent.

In said agent for promoting percutaneous absorption, the above-mentioned oxidized coenzyme Q or the above-mentioned reduced coenzyme Q may be independently used as coenzyme Q. Moreover, the above-mentioned oxidized coenzyme Q and the above-mentioned reduced coenzyme Q may be used in combination. In this case, the content ratio of reduced coenzyme Q is preferably 60% by weight or more, and more preferably 80% by weight or more in the total amount of coenzyme Q.

The agent for promoting percutaneous absorption containing the above-mentioned coenzyme Q shows a percutaneous absorption promoting effect to an antifungal agent, and is not irritative to skin.

The composition for percutaneous administration of the present invention comprises the above-mentioned agent for promoting percutaneous absorption. Moreover, when a drug or other component is added to said composition, said drug and other component are not restricted provided that those aiming at percutaneous administration.

As the above-mentioned drug, there may be mentioned the below-mentioned antifungal agent, and the like. Additionally, as the other component mentioned above, there may be mentioned, for example, moisturizers (e.g. hyaluronic acid, collagen, etc.), antibacterial agents, antipruritic ingredients, and the like.

Moreover, said composition for percutaneous administration may contain medically, and the like, acceptable various additives other than coenzyme Q. As said various additives, there may be mentioned, for example, preservatives, disinfectants, perfumes, foaming inhibitors, colorants, pigments having a coloring action, thickeners, surfactants, emulsifiers, softening agents, humidifying agents and/or moisturizers, fats, oils, waxes, alcohols, polyols, polymers, bubble stabilizers, electrolytes, organic solvents, silicone derivatives, etc.

Furthermore, an antioxidant, a health food material, nutritional supplement material, vitamin, absorption promoter, etc. other than the above-mentioned additives may also be contained.

The form of said composition for percutaneous administration is not particularly restricted. But for example, there may be mentioned one obtainable by dissolving, or mixing and dispersing the above-mentioned drug in an appropriate base to prepare cream, paste, jelly, gel, emulsion, and liquid (e.g. ointment, liniment, lotion or spray); one obtainable by dissolving, or mixing and dispersing the above-mentioned drug in a base, and flatting the resultant on a support (e.g. catalpasm); one obtainable by dissolving, or mixing and dispersing the above-mentioned drug in an adhesive, and flatting the resultant on a support (e.g. plaster, tape); and the like.

Said composition for percutaneous administration may be contained in, for example, a dermatological drug for external application, cosmetics, adhesive preparation, matrix for adhesive preparation, bath agent, cleanser, etc. prepared for treating athlete's foot.

In the composition for percutaneous administration of the present invention, reduced type and oxidized type of coenzyme Q may be used independently, or in combination. The content of coenzyme Q in the composition is preferably 0.001 to 20% by weight, and more preferably 0.01 to 5% by weight relative to the total amount of the composition.

Moreover, the composition for treating athlete's foot of the present invention comprises the above-mentioned agent for promoting percutaneous absorption and an antifungal agent.

As the antifungal agent to be used, there is not any restriction provided that those used in general. As an antifungal agent which exerts its effect better in combination with the above-mentioned agent for promoting percutaneous absorption, there may be mentioned but is not limited to, for example, imidazole antifungal agents (econazole, iconazole, bifonazole, ketoconazole, etc.), clotrimazole, miconazole, fluconazole, and the like. Preferred are imidazole antifungal agents such as econazole.

The content of said antifungal agent is hot particularly restricted, and for example, an effective amount for treating athlete's foot may be contained.

Moreover, the dermatologic treatment method of the present invention comprises combinedly using the above-mentioned composition for percutaneous administration and a dermatologic composition.

As the dermatologic treatment method, for example, it is also possible to combinedly use the composition for percutaneous administration containing coenzyme Q such as ointment, and other dermatologic composition (e.g. ointment for athlete's foot, antipsoriatic agent, etc.). In this case, if both are ointments, the percutaneous absorption promoting effect obtainable by the present invention can be expected by applying them in layers.

### BEST MODE FOR CARRYING OUT THE INVENTION

In the following, the present invention is described in further detail by way of examples. However, these examples are not limitative of the present invention.

### (Example 1) Percutaneous absorption promoting effect of reduced coenzyme Q₁₀ and oxidized coenzyme Q₁₀ to an antifungal agent

Using clotrimazole, bifonazole, and econazole as antifungal agents, the percutaneous absorption promoting effects of reduced coenzyme Q₁₀ and oxidized coenzyme Q₁₀ to these antifungal agents were evaluated. The antifungal agents were separately dissolved in ethanol, mixed with heat-melted polyethylene glycol 1000 (PEG 1000) so as to have the final concentration of 1%, and prepared into ointments by allowing them to stand at room temperature. Each of heat-melted reduced coenzyme Q₁₀ and oxidized coenzyme Q₁₀ was mixed with the above-mentioned heat-melted PEG 1000 ointment containing each antifungal agent to prepare mixed ointments in which each of the final concentrations of coenzyme Q₁₀s and antifungal agent is 1%.

A percutaneous absorbability test was carried out using fully fed male hairless rats (weight: 350 to 400 g). The hairless rats were mildly anesthetized with ether, and three administration parts of 3 cm square were set on the backs of each rat. To each part, 0.1 g of each ointment, i.e. the ointment containing an antifungal agent alone, the ointment containing an antifungal agent and reduced coenzyme Q₁₀, and the ointment containing an antifungal agent and oxidized coenzyme Q₁₀ was administered. After the lapse of 2 hours from the administration, the rat was euthanized, the administration parts were thoroughly washed, and skin was collected. The clotrimazole amounts in skin were determined by the sequential treatment subjecting the skin to homogenization, acetone extraction, concentration using a solid phase column, and high performance liquid chromatography. Each amount of bifonazole and econazole in the skin was determined by the sequential treatment subjecting the skin to homogenization, methanol extraction, and high performance liquid chromatography.

The results are shown in Table 1. The values are the average value ± standard deviation (n = 3). By the results of this test, it was made clear that oxidized coenzyme Q₁₀ and reduced coenzyme Q₁₀ have a percutaneous absorption promoting effect to an antifungal agent. The antifungal agent amount in skin increased approximately 20 to 40% by oxidized coenzyme Q₁₀. In addition, the percutaneous absorption promoting effect of reduced coenzyme Q₁₀ was higher than that of oxidized coenzyme Q₁₀, and the antifungal agent amount in skin increased approximately 20 to 70%. Having shown the percutaneous absorption promoting effect nearly evenly to three typical antifungal agents, it was shown that oxidized coenzyme Q₁₀ and reduced coenzyme Q₁₀ have a wide availability as agents for promoting percutaneous absorption to an antifungal agent.

**Table 1**

| | Amount of antifungal agent in skin (µg/g) | | |
|---|---|---|---|
| | Clotrimazole | Bifonazole | Econazole |
| Antifungal agent alone | 72.7±5.7 | 1.86±0.08 | 3.11±0.96 |
| | (100) | (100) | (100) |
| Antifungal agent + oxidized coenzyme Q₁₀ | 104.5±13.4 | 2.23±0.19 | 3.89±0.72 |
| | (144**) | (120*) | (125) |
| Antifungal agent + reduced coenzyme Q₁₀ | 89.8±9.3 | 2.88±0.67 | 5.23±0.61 |
| | (124**) | (154**) | (168**) |
| Reduced coenzyme Q₁₀ contains 5% of oxidized coenzyme Q₁₀. | | | |

| | | | |
|---|---|---|---|
| *p<0.05, | | | |
| **p<0.01 Student t-test | | | |

### (Example 2) Skin irritation test

A skin irritation test was carried out using JW rabbits (17 to 18 weeks old). The backs of three rabbits were dehaired by an electric hair clipper, and four divisions of administration parts of 2.5 x 2.5 cm square were set on each rabbit back. 0.5 g of each specimen, i.e. oxidized coenzyme Q₁₀ and reduced coenzyme Q₁₀ (containing 2% of oxidized coenzyme Q₁₀) was administered on the administration parts, and the parts were covered with gauzes for specimen's fixation. After the lapse of 4 hours, the specimens were removed. Further after the lapse of 1 hour, 24 hours, 48 hours, and 72 hours, formation of erythema, crusta and dropsy was determined by the method according to Draize J. H. et al. (Exp. Ther., 82, 377-390, 1944). That is, observations were made in view of formations of erythema, crusta and dropsy, they were scored for calculation of the primary irritation indexes, and the skin irritations were evaluated based on said primary irritation indexes. As a result, all scores were 0 (i.e. no erythema, crusta or dropsy) in observation items of oxidized coenzyme Q₁₀ and reduced coenzyme Q₁₀. Therefore, no skin irritation was observed in these substances.

### INDUSTRIAL APPLICABILITY

The coenzyme Q-containing agent for promoting percutaneous absorption of the present invention shows a percutaneous absorption promoting effect to an antifungal agent, and is not irritative to skin, thus can provide a highly useful composition for treating athlete's foot.

## Claims

1. An agent for promoting percutaneous absorption which comprises oxidized coenzyme Q represented by the following formula (1) 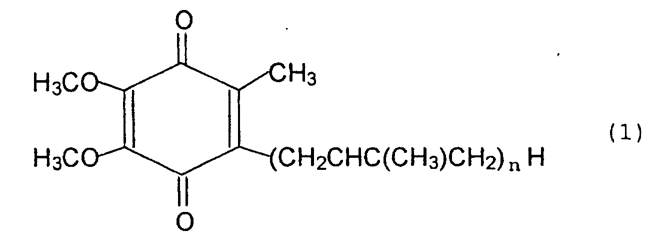 (wherein n represents an integer of 1 to 12) and/or reduced coenzyme Q represented by the following formula (2) 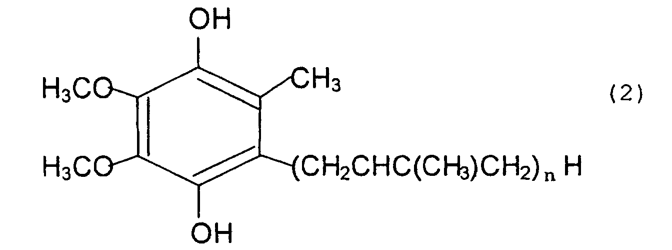 (wherein n represents an integer of 1 to 12) as an active ingredient.

2. The agent for promoting percutaneous absorption according to Claim 1,
wherein coenzyme Q is coenzyme Q₁₀.

3. A composition for percutaneous administration
which comprises the agent for promoting percutaneous absorption according to Claim 1 or 2.

4. A composition for treating athlete's foot
which comprises the agent for promoting percutaneous absorption according to Claim 1 or 2, and an antifungal agent.

5. A dermatologic treatment method
which comprises combinedly using the composition for percutaneous administration according to Claim 3 and a dermatologic composition.
